# EUROPÄISCHE PATENTSCHRIFT

(11) **EP 3 310 273 B1**
(45) Veröffentlichungstag und Bekanntmachung des Hinweises auf die Patenterteilung: **30.09.2020**
(21) Anmeldenummer: 16741851.6
(22) Anmeldetag: 09.06.2016
(51) Int. Cl.: A61B 17/16, A61B 17/88, A61B 17/92

(54) **CHIRURGISCHER INSTRUMENTENSATZ**
SURGICAL INSTRUMENT KIT
INSTRUMENT CHIRURGICAL

(30) Priorität: 16.06.2015 DE 102015007540
(43) Veröffentlichungstag der Anmeldung: 25.04.2018
(73) Patentinhaber: REINHARD Feinmechanik GmbH, 63128 Dietzenbach (DE); Missalla, Adalbert, 61440 Oberursel (DE)
(72) Erfinder: MISSALLA, Adalbert, 61440 Oberursel (DE); REINHARD, Helmut, 60386 Frankfurt (DE)
(74) Vertreter: Oppermann, Mark
(86) Internationale Anmeldenummer: PCT/EP2016/000944
(87) Internationale Veröffentlichungsnummer: WO 2016/202441

(56) Entgegenhaltungen:
- WO-A1-2005/055845
- US-A- 5 906 210
- US-A1- 2005 240 197
- US-A1- 2008 269 756

## Beschreibung

### TECHNISCHES GEBIET

Die vorliegende Erfindung bezieht sich auf einen chirurgischen Instrumentensatz, der wenigstens ein stabförmiges Treibwerkzeug und eine Impulsmasse zum Aufbringen von äußeren Kräften am Treibwerkzeug aufweist. Derartige Instrumentensätze kommen zum Einsatz, wenn es darum geht, ein Volumen - wie etwa einen Marknagel oder ein Prothesenteil - in einen Knochen axial ein- bzw. auszutreiben. Insbesondere finden solche Instrumentensätze aber auch in der rekonstruktiven Chirurgie bei der autologen Wiederherstellung von Kreuzbändern Verwendung, wobei sie dazu dienen, Knochenbohrungen für die Erstellung von Implantatlagern kontrolliert aufzuweiten.

### STAND DER TECHNIK

Bei dem hier betrachteten Anwendungsbeispiel des chirurgischen Instrumentensatzes im Zusammenhang mit der autologen Wiederherstellung des vorderen Kreuzbandes nach der sogenannten "All-Press-Fit"-Technik - die als fremdkörperfreie Operationsmethode mit biologischer und gelenknaher Einheilung des Sehnenimplantats gegenüber konventionellen Verfahren mit körperfremden Befestigungsmitteln besonders vorteilhaft ist - wird üblicherweise kurzgefasst wie folgt vorgegangen:
In einem ersten Operationsschritt wird dem Patienten Sehnenmaterial (von der Semitendinosus und ggf. zusätzlich der Gracilissehne) entnommen, welches später zu einem Kreuzband-Sehnenimplantat weiterverarbeitet wird. Die Fixierung des Sehnenimplantats erfolgt, wie nachfolgend näher beschrieben wird, doppelt femoral und tibial über je eine Knochenbrücke und zusätzlich mit kortikospongiösen Knochenzylindern. Diese Knochenzylinder werden aus femoralen und tibialen Knochenbohrungen gewonnen.

Genauer gesagt werden eine durchgehende obere tibiale Knochenbohrung und eine femorale Knochenbohrung in der Art eines Sacklochs sowie eine weitere untere tibiale Knochenbohrung, ebenfalls in der Art eines Sacklochs, erzeugt. Die beiden tibialen Knochenbohrungen werden zur Erstellung der tibialen Knochenbrücke mittels eines kleinen Verbindungskanals verbunden. Am proximalen Ende der femoralen Knochenbohrung werden zwei kleine, die femorale Knochenbohrung parallel zu deren Mittelachse verlängernde und durchgehende Fadenkanäle für die femorale Knochenbrücke erzeugt. Die Erstellung der drei erstgenannten Knochenbohrungen erfolgt z.B. mit Hilfe von Diamanthohlschleifen, wobei die gewonnenen Bohrkerne (Knochenzylinder) später zur Verankerung des Sehnenimplantats in den Knochenbohrungen und zum Abschluss der tibialen Knochenbohrungen verwendet werden.

Zur Erstellung des ca. 7 cm langen Sehnenimplantats wird das entnommene Sehnenmaterial - nach Bestimmung der Sehnenstärke mit Hilfe einer Lehre, mittels der ein äquivalenter Sehnendurchmesser ermittelt wird - an den Enden aneinandergenäht, zu einer Schlaufe umgeschlagen und dadurch doppelt verwendet. Proximal wird das Sehnenimplantat von zwei Fäden gefasst; distal wird der aus der unteren Knochenbohrung der Tibia gewonnene, ca. 15 mm lange Knochenzylinder eingenäht. Das Sehnenimplantat wird hierbei standardisiert mit proximalen und distalen Fäden zur Sicherung über den Knochenbrücken auf einem speziell für diese Technik entwickelten Arbeitsbrett vorbereitet, das in der Druckschrift DE 20 2014 007 804 U1 näher beschrieben ist.

Da die Stärke, d.h. der Querschnitt des verwendeten Sehnenmaterials von Patient zu Patient unterschiedlich groß ist, während die Knochenzylinder mit standardisierten Werkzeugen, z.B. den vorerwähnten Diamanthohlschleifen gewonnen werden und somit definierte Durchmesser besitzen, ist das vorbereitete Sehnenimplantat an seinen in den Knochenbohrungen aufzunehmenden Enden im Querschnitt individuell verschieden groß. Auch deshalb ist die femorale wie auch die obere tibiale Knochenbohrung zusätzlich aufzuweiten.

Hierfür kommen als stabförmige Treibwerkzeuge ausgebildete Dilatatoren zum Einsatz, die einen Schaft mit einem ersten Ende und einem zweiten Ende aufweisen, wobei das erste Ende einen bezüglich der Mittelachse des Dilatators symmetrischen oder asymmetrischen Aufweitungsquerschnitt besitzt. Dilatatoren mit einem asymmetrischen Aufweitungsquerschnitt werden insbesondere bei großen Sehnenstärken eingesetzt, um ein im Querschnitt gesehen tangential an die Knochenbohrung anschließendes Sehnenlager auszubilden.

Die Aufweitung der Knochenbohrungen erfolgt in mehreren, z.B. bis zu 8 Dilatationsstufen pro Knochenbohrung, um eine Überlastung des Knochens zu vermeiden. Mit von Dilatator zu Dilatator zunehmendem Aufweitungsquerschnitt werden die Dilatatoren durch Aufbringung äußerer Kräfte am zweiten Ende des Schafts mittels eines Hammers in die jeweilige Knochenbohrung eingeschlagen und anschließend wieder unter Aufbringung einer entgegengesetzten äußeren Kraft am zweiten Ende des Schafts aus der entsprechenden Knochenbohrung herausgetrieben. Die Kraft zum Austreiben des Dilatators wird durch eine um den Schaft des Dilatators gelegte Impulsmasse aufgebracht, die gegen eine Anschlagfläche am zweiten Ende des Dilatators von der Knochenbohrung weg beschleunigt wird.

Nach Ausbildung der Implantatlager wird das vorbereitete Sehnenimplantat mittels der proximalen Fäden, welche vorher durch die die femorale Knochenbohrung verlängernden Fadenkanäle hindurchgezogen wurden, durch die geeignet aufgeweitete, obere tibiale Knochenbohrung bis vor die geeignet aufgeweitete, femorale Knochenbohrung eingezogen. Vor dem kompletten Einzug in die femorale Knochenbohrung wird das Sehnenimplantat an dem in der oberen tibialen Knochenbohrung aufzunehmenden Ende um 90° lateral gedreht. Dadurch werden die Implantatanteile so platziert, dass ein anteromediales und ein posterolaterales Bündel entstehen, wodurch die anatomische Form des natürlichen Kreuzbandes nachgebildet wird. Das Sehnenimplantat wird nun 15 mm tief in die femorale Knochenbohrung eingezogen (und dabei teilweise tibial eingeschlagen) und sodann von oberhalb des Femurs mittels der proximalen Fäden gespannt, worauf der aus der Knochenbohrung des Femurs gewonnene, geeignet gekürzte Knochenzylinder mittels eines Applikators - umfassend ebenfalls ein stabförmiges Treibwerkzeug und eine Führung für den Knochenzylinder - in die aufgeweitete femorale Knochenbohrung zur Erzeugung der "Press-Fit"-Verbindung eingetrieben und somit fixiert wird.

Nach Streckung des Kniegelenks erfolgt die an die individuelle Geometrie des Gelenks selbstadaptierende Anpassung der Kreuzbandspannung. Die unteren der distalen Fäden des Implantats werden von der oberen tibialen Knochenbohrung durch den senkrechten Verbindungskanal in die untere tibiale Knochenbohrung gezogen und sodann mit den oberen der distalen Fäden aus der oberen tibialen Knochenbohrung verknotet (Knochenbrücke). Abschließend werden die Knochenbohrungen in der Tibia mit Abschnitten des aus der oberen Knochenbohrung der Tibia gewonnenen Knochenzylinders verblockt, worauf die beiden proximalen Fäden des Implantats über der femoralen Knochenbrücke verknotet werden.

Wie aus dem oben beschriebenen Ablauf ersichtlich erfolgt die Aufweitung einer Knochenbohrung in bis zu 8 Dilatationsstufen. Dabei erfordert schon eine Dilatationsstufe eine Mehrzahl von Handreichungen zwischen dem Operateur und einem oder mehreren Assistenten; so müssen dem Operateur für einen reibungslosen Operationsablauf ein Dilatator in der korrekten Abmessung sowie im Wechsel der Hammer zum Eintreiben des Dilatators und die Impulsmasse zum Austreiben des Dilatators angereicht werden. Bei mehreren Dilatationsstufen sind also eine Vielzahl von Handreichungen notwendig, wobei auch auf die richtige Reihenfolge der Dilatatoren zu achten ist. Je mehr Handreichungen erforderlich sind, desto größer ist indes die Gefahr, dass Fehler oder Probleme bei der Übergabe auftreten.

Ein chirurgischer Instrumentensatz, der die Merkmale des Oberbegriffs des Patentanspruchs 1 aufweist, ist aus der Druckschrift US 5,906,210 bekannt.

### AUFGABENSTELLUNG

Der Erfindung liegt ausgehend von dem oben geschilderten Stand der Technik die Aufgabe zugrunde, einen chirurgischen Instrumentensatz bereitzustellen, der insbesondere in der rekonstruktiven Chirurgie zur Aufweitung von Knochenbohrungen einsetzbar ist und dabei eine Optimierung des Arbeitsablaufs ermöglicht.

### DARSTELLUNG DER ERFINDUNG

Diese Aufgabe wird durch die im Patentanspruch 1 angegebenen Merkmale gelöst. Vorteilhafte bzw. zweckmäßige Weiterbildungen der Erfindung sind Gegenstand der Patentansprüche 2 bis 12.

Bei einem chirurgischen Instrumentensatz, umfassend mindestens ein stabförmiges Treibwerkzeug, das einen Schaft mit einem ersten Ende und einem zweiten Ende aufweist, welches zur Einleitung von äußeren Kräften in Richtung des ersten Endes oder davon weg ausgebildet ist, und eine Impulsmasse zum Aufbringen der äußeren Kräfte am zweiten Ende, wobei das zweite Ende und die Impulsmasse jeweils eine Kraftübertragungsfläche besitzen, die zusammen eine Flächenpaarung bilden, über die die äußeren Kräfte in Richtung des ersten Endes einleitbar sind, sowie mit komplementären Strukturen versehen sind, über welche die äußeren Kräfte in Richtung weg vom ersten Ende aufbringbar sind, wobei die komplementären Strukturen eine dem ersten Ende zugewandte Einleitungsfläche am zweiten Ende des Schafts des Treibwerkzeugs und eine damit zur Krafteinleitung schlagend in Anlage bringbare Gegenfläche an der Impulsmasse aufweisen, wobei die Impulsmasse die Form eines Hammers aufweist, mit einem Hammerkopf, dessen Mittelachse eine erste Achse der Impulsmasse definiert, und einem Hammerstiel, dessen Mittelachse eine von der ersten Achse verschiedene zweite Achse der Impulsmasse definiert, und wobei die Kraftübertragungsfläche für die äußeren Kräfte in Richtung des ersten Endes senkrecht zur ersten Achse ausgerichtet ist; ist erfindungsgemäß die Gegenfläche für die äußeren Kräfte in Richtung weg vom ersten Ende senkrecht zur zweiten Achse ausgerichtet.

Im erfindungsgemäßen Instrumentensatz ist also nur ein Werkzeug zum Aufbringen der äußeren Kräfte am Treibwerkzeug, nämlich die Impulsmasse vorgesehen, mittels der am zweiten Ende des Treibwerkzeugs die äußeren Kräfte sowohl vom zweiten Ende des Treibwerkzeugs in Richtung des ersten Endes des Treibwerkzeugs - über die Flächenpaarung - als auch in der Gegenrichtung - über die komplementären Strukturen - aufgebracht werden können. Da der Operateur im Ergebnis die erfindungsgemäße Impulsmasse in der Hand behalten kann, entfallen etwa im Falle der oben beschriebenen autologen Kreuzbandrekonstruktion pro aufzuweitender Knochenbohrung die den Arbeitsfluss unterbrechenden, bis zu 16 Handreichungen für die krafteinleitenden Werkzeuge, die diesbezügliche Kommunikation zwischen Operateur und Assistent wie auch die hierfür erforderlichen Zeiten. So werden etwaige Übergabeprobleme oder Falschwechsel vermieden, der Operateur kann besser "bei der Sache" bleiben und die Fehlerwahrscheinlichkeit nimmt insgesamt ab. Dabei ergibt sich eine signifikante Verkürzung der gesamten Operationszeit, was nicht nur für den Patienten von Vorteil ist, sondern auch zu einer Kostenreduktion führt.

Dass die komplementären Strukturen die dem ersten Ende zugewandte Einleitungsfläche am zweiten Ende des Schafts des Treibwerkzeugs und die damit zur Krafteinleitung schlagend in Anlage bringbare Gegenfläche an der Impulsmasse aufweisen, hat den Vorteil, dass für die Impulsmasse ein maximaler Beschleunigungsweg zur Verfügung steht, bevor deren Gegenfläche schlagend mit der Einleitungsfläche des Treibwerkzeugs zur Anlage gelangt. Außerdem bedingt das beanspruchte Vorsehen der Einleitungsfläche am Schaft eine geringe Komplexität und eine gute Reinigungsmöglichkeit des Treibwerkzeugs.

Für den Operateur ist ferner die beanspruchte Hammerform der Impulsmasse besonders vorteilhaft, bei der die Kraftübertragungsfläche für die äußeren Kräfte in Richtung des ersten Endes (Eintreiben) senkrecht zur Mittelachse des Hammerkopfs ausgerichtet ist, während die Gegenfläche für die äußeren Kräfte in Richtung weg vom ersten Ende (Austreiben) senkrecht zur Mittelachse des Hammerstiels ausgerichtet ist, weil er sich nicht an ein neues Einschlagwerkzeug gewöhnen muss, sondern mit dem ihm vom Handling bereits vertrauten Hammer arbeiten kann. Beim Austreiben des Treibwerkzeugs dient der Hammerstiel, der mit der Achse des Treibwerkzeugs ausgerichtet wird, zudem als Orientierungshilfe, wobei der Operateur zur möglichst ungebremsten Übertragung des Austreibimpulses ohne Kontrollverlust die Hammerführungshand leicht öffnen kann, was auch ein Ausrichten der komplementären Strukturen ermöglicht und somit der Entstehung von Querkräften entgegenwirkt.

Prinzipiell ist es möglich, im Schaft des Treibwerkzeugs einen Längsschlitz mit der dem ersten Ende zugewandten Einleitungsfläche auszubilden, gegen die die Impulsmasse mit ihrer Gegenfläche beschleunigt werden kann, um am Treibwerkzeug die äußeren Kräfte in Richtung weg vom ersten Ende aufzubringen. Im Hinblick auf eine demgegenüber wiederum geringere Komplexität und bessere Reinigungsmöglichkeit ist es indes bevorzugt, wenn die dem ersten Ende zugewandte Einleitungsfläche am zweiten Ende des Schafts gegenüber einem sich zwischen den Enden des Schafts erstreckenden Mittelabschnitt des Schafts radial übersteht. Gegenüber der vorerwähnten Schlitzlösung ist diese Ausgestaltung auch insofern vorteilhaft, als der Schaft nicht geschwächt wird und keine Verletzungsgefahr besteht.

Hierbei ist die dem ersten Ende zugewandte Einleitungsfläche am zweiten Ende des Schafts vorzugsweise eine Ringfläche. Gegenüber ebenfalls denkbaren Ausgestaltungen, wie etwa einem Anschlagstift oder einzelnen Vorsprüngen, die symmetrisch oder unsymmetrisch am Umfang des Schafts verteilt angeordnet sein können, hat die Ringfläche den Vorteil, dass es aufgrund der im Verhältnis großen umlaufenden Kontaktfläche bei der Kraftübertragung zu einer nur geringen Flächenpressung kommt, was die Gefahr einer Beschädigung reduziert. Darüber hinaus ist eine Ringfläche vorteilhaft, weil die Impulsmasse bezüglich des Treibwerkzeugs nicht im Drehwinkel um dessen Mittelachse orientiert werden muss.

Grundsätzlich kann die dem ersten Ende zugewandte Einleitungsfläche am zweiten Ende des Schafts senkrecht zur Längsachse des Schafts oder entlang des Schafts konisch bzw. kegelig ausgebildet sein. Demgegenüber bevorzugt ist allerdings eine Ausbildung, bei der die dem ersten Ende zugewandte Einleitungsfläche am zweiten Ende des Schafts ballig ausgestaltet ist. Eine solche Rundung erleichtert das Fügen der Impulsmasse und sorgt für eine gute Haptik des Treibwerkzeugs. Darüber hinaus ist die ballige Ausgestaltung im Hinblick auf die Vermeidung von Querkräften von Vorteil; die äußeren Kräfte werden auch bei einer Winkelfehlstellung der in Längsrichtung des Schafts beschleunigten Impulsmasse mit deren Anschlag an der Einleitungsfläche in einer bezüglich der Längsachse des Schafts im Wesentlichen axialen Richtung eingeleitet.

In einer besonders einfachen Ausgestaltung kann die Kraftübertragungsfläche zur Einleitung der äußeren Kräfte in Richtung des ersten Endes des Schafts, d.h. zum Eintreiben des Treibwerkzeugs senkrecht zu dessen Längsachse stehen, so dass diese die Flächennormale der Kraftübertragungsfläche bildet. Hierbei können im Falle einer außermittigen Krafteinleitung infolge eines exzentrischen Auftreffens der Impulsmasse auf der Kraftübertragungsfläche des Treibwerkzeugs aber unerwünschte Querkräfte entstehen. In einer bevorzugten Ausgestaltung des Treibwerkzeugs wird dem dadurch begegnet, dass das zweite Ende des Schafts einen kugelkalottenförmigen Endabschnitt aufweist, der die Kraftübertragungsfläche zur Einleitung der äußeren Kräfte in Richtung des ersten Endes des Schafts ausbildet. Eine vornehmlich in einer Richtung entlang des Schafts zu dessen ersten Ende hin beschleunigte Impulsmasse trifft den Schaft mit ihrer Kraftübertragungsfläche stets an der "vorstehenden" Kugelkalotte und somit im Bereich der Längsachse des Schafts.

Im weiteren Verfolg des Erfindungsgedankens besitzt die Impulsmasse zur Aufnahme des Mittelabschnitts des Schafts vorzugsweise eine Aussparung, die eine die Gegenfläche an der Impulsmasse - innerhalb oder außerhalb der Aussparung - ausbildende Schulter aufweist, an der die dem ersten Ende zugewandte, radial überstehende Einleitungsfläche am zweiten Ende des Schafts schlagend in Anlage bringbar ist. Gegenüber einer ebenfalls denkbaren Alternative, bei der die Impulsmasse geeignet gabelförmig gestaltet ist, um unter bezüglich des Schafts seitlicher Kraftaufbringung mit der radial überstehenden Einleitungsfläche am Treibwerkzeug in Anlage zu gelangen, hat die bevorzugte Ausbildung mit Aussparung zur Aufnahme des Mittelabschnitts des Schafts und Anschlagschulter den Vorteil, dass die Impulsmasse bezüglich der Längsachse des Schafts zentriert sowie entlang des Schafts zielgerichtet axial - im Gegensatz zu einer bogenförmigen Bahn - beschleunigt werden kann, so dass die Impulsmasse mit ihrer Gegenfläche im Wesentlichen ohne Einbringung von Querkräften an der zugeordneten Einleitungsfläche des Treibwerkzeugs anschlagen kann, um das Treibwerkzeug auszutreiben.

Hierbei ist die Impulsmasse vorzugsweise mit einem Schlitz versehen, der parallel zu der Aussparung verläuft und über den der Mittelabschnitt des Schafts in die Aussparung einführbar ist. Dies ermöglicht auf einfache Weise ein seitliches Fügen der Impulsmasse zum eingetriebenen Treibwerkzeug.

In einer besonders bevorzugten Ausgestaltung der Impulsmasse ist ferner die Gegenfläche an der Schulter auf einer bezüglich der Aussparung inneren Seite der Schulter ausgebildet und die Aussparung weist vor der Schulter einen lichten Querschnitt auf, der für ein ungehindertes Bewegen auch des zweiten Endes des Schafts in der Aussparung größer ist als der größte Querschnitt des zweiten Endes. Somit wird auf einfache Weise bei einer Beschleunigung der Impulsmasse entlang des Schafts für eine - zweckmäßig mit einem gewissen Spiel versehene - Führung der Impulsmasse am in der Aussparung befindlichen zweiten Ende des Treibwerkzeugs Sorge getragen, so dass die Gefahr gebannt ist, dass die Impulsmasse mit ihrer aussparungsinneren Gegenfläche nicht zentrisch auf der Einleitungsfläche des Treibwerkzeugs auftrifft oder gar von dem Schaft des Treibwerkzeugs abgehoben wird. Außerdem kann die Impulsmasse beim Beschleunigen entlang des Treibwerkzeugs über das zweite Ende des Schafts hinaus bewegt werden, um mit ihrer an der Schulter ausgebildeten Gegenfläche an der zugeordneten Einleitungsfläche des Treibwerkzeugs in Anlage zu kommen, was die vorerwähnte vorteilhafte Führung der Impulsmasse auch bei kurzer, mithin während der Operation gut handhabbarer Schaftlänge ermöglicht.

Die Ausgestaltung der Aussparung der Impulsmasse kann grundsätzlich so getroffen sein, dass sich die Aussparung ausschließlich im Hammerstiel befindet und vor dem Hammerkopf endet. Insbesondere im Hinblick auf kompakte Abmessungen von Treibwerkzeug und Impulsmasse bevorzugt ist es jedoch, wenn die Aussparung zur Aufnahme des Schafts des Treibwerkzeugs mit der zweiten Achse der Impulsmasse ausgefluchtet ist und sich vom Hammerstiel in den Hammerkopf hinein erstreckt, wo die Aussparung mit ihrer Schulter offen endet. Bildet ein spiegelsymmetrisch ausgestalteter Hammerkopf die größte Teilmasse der Impulsmasse, hat das Vorsehen der Schulter mit der schlagend in Anlage zu bringenden Gegenfläche im Hammerkopf ferner den Vorteil, dass die Übertragung des Austreibimpulses nahe dem Massenschwerpunkt der Impulsmasse erfolgt, so dass keine größeren Momente um die Gegenfläche entstehen.

Prinzipiell ist es möglich, die Ausgestaltung und Anordnung der Aussparung im Hammerstiel so zu treffen, dass das zweite Ende des Schafts über ein offenes Ende des Hammerstiels in die Aussparung eingeführt werden kann. Vorzugsweise weist der Hammerstiel jedoch eine sich in Richtung quer zur zweiten Achse erstreckende und an den Schlitz anschließende Fügeöffnung auf, die in der Aussparung endet und dazu dient, das zweite Ende des Schafts in die Aussparung einzuführen. Durch das Vorsehen einer Fügeöffnung kann die Länge des Schafts des Eintreibwerkzeugs vorteilhaft unabhängig von der Länge des Hammerstiels den jeweiligen Erfordernissen entsprechend optimal festgelegt werden, und umgekehrt.

Für die Schlagfläche(n) am Hammerkopf sind grundsätzlich verschiedene Anordnungen und Ausgestaltungen denkbar. So kann etwa nur eine Schlagfläche auf einer Seite des Hammerstiels am Hammerkopf vorgesehen sein. Die Schlagfläche selbst kann ferner ballig oder in anderer Art und Weise gekrümmt sein. Insbesondere im Hinblick auf eine ergonomische Handhabung (zu Schlagflächenanzahl und -anordnung) und Querkraftfreiheit (zu Schlagflächenform) bevorzugt ist allerdings eine - idealerweise symmetrische - Ausgestaltung, bei der der Hammerkopf auf voneinander abgewandten Seiten jeweils eine ebene Schlagfläche als mögliche Kraftübertragungsfläche aufweist.

Schließlich kann das Treibwerkzeug für eine hier bevorzugte Anwendung des Instrumentensatzes in z.B. der rekonstruktiven Chirurgie bei der Wiederherstellung von Bändern ein Dilatator zum Aufweiten einer Knochenbohrung sein, der an seinem ersten Ende einen für eine symmetrische oder asymmetrische Aufweitung der Knochenbohrung ausgestalteten Aufweitungsabschnitt aufweist.

### KURZE BESCHREIBUNG DER ZEICHNUNGEN

Im Folgenden wird die Erfindung anhand eines bevorzugten Ausführungsbeispiels unter Bezugnahme auf die beigefügten, teilweise schematischen bzw. vereinfachten Zeichnungen näher erläutert. In den Zeichnungen zeigen:
- Fig. 1: eine perspektivische Darstellung eines chirurgischen Instrumentensatzes nach einem bevorzugten Ausführungsbeispiel der Erfindung, bestehend aus wenigstens einem, hier zwei stabförmigen Treibwerkzeugen in Form von Dilatatoren zur symmetrischen und zur asymmetrischen Aufweitung von Knochenbohrungen, und einer Impulsmasse in Form eines Hammers zum Ein- und Austreiben der Dilatatoren;
- Fig. 2: eine Draufsicht auf den Hammer des chirurgischen Instrumentensatzes gemäß Fig. 1;
- Fig. 3: eine Schnittansicht des Hammers gemäß Fig. 1 entsprechend der Schnittverlaufslinie III-III in Fig. 2;
- Fig. 4: eine im Maßstab gegenüber der Darstellung in Fig. 2 vergrößerte, seitlich abgebrochene Schnittansicht des Hammers gemäß Fig. 1 entsprechend der Schnittverlaufslinie IV-IV in Fig. 2;
- Fig. 5: eine im Maßstab gegenüber der Darstellung in Fig. 2 vergrößerte, seitlich abgebrochene Schnittansicht des Hammers gemäß Fig. 1 entsprechend der Schnittverlaufslinie V-V in Fig. 2;
- Fig. 6: eine Draufsicht auf den für die symmetrische Aufweitung vorgesehenen Dilatator des chirurgischen Instrumentensatzes gemäß Fig. 1;
- Fig. 7: eine Schnittansicht des Dilatators zur symmetrischen Aufweitung gemäß Fig. 1 entsprechend der Schnittverlaufslinie VII-VII in Fig. 6;
- Fig. 8: eine Draufsicht auf den für die asymmetrische Aufweitung vorgesehenen Dilatator des chirurgischen Instrumentensatzes gemäß Fig. 1;
- Fig. 9: eine Schnittansicht des Dilatators zur asymmetrischen Aufweitung gemäß Fig. 1 entsprechend der Schnittverlaufslinie IX-IX in Fig. 8;
- Fig. 10: eine Schnittansicht des Dilatators zur asymmetrischen Aufweitung gemäß Fig. 1 entsprechend der Schnittverlaufslinie X-X in Fig. 8;
- Fig. 11: eine Prinzipskizze mit dem Hammer und dem Dilatator zur symmetrischen Aufweitung von Fig. 1, die veranschaulicht, wie der Dilatator mittels des Hammers über eine Flächenpaarung an Dilatator und Hammer in die Knochenbohrung eingetrieben wird;
- Fig. 12: eine Prinzipskizze mit dem Hammer und dem Dilatator zur symmetrischen Aufweitung von Fig. 1, die veranschaulicht, wie der Dilatator mittels des Hammers über komplementäre Strukturen an Dilatator und Hammer aus der Knochenbohrung ausgetrieben wird;
- Fig. 13: eine Prinzipskizze mit dem Hammer und dem Dilatator zur asymmetrischen Aufweitung von Fig. 1, die veranschaulicht, wie der Dilatator mittels des Hammers über eine Flächenpaarung an Dilatator und Hammer in die Knochenbohrung eingetrieben wird; und
- Fig. 14: eine Prinzipskizze mit dem Hammer und dem Dilatator zur asymmetrischen Aufweitung von Fig. 1, die veranschaulicht, wie der Dilatator mittels des Hammers über komplementäre Strukturen an Dilatator und Hammer aus der Knochenbohrung ausgetrieben wird.

### DETAILLIERTE BESCHREIBUNG DES AUSFÜHRUNGSBEISPIELS

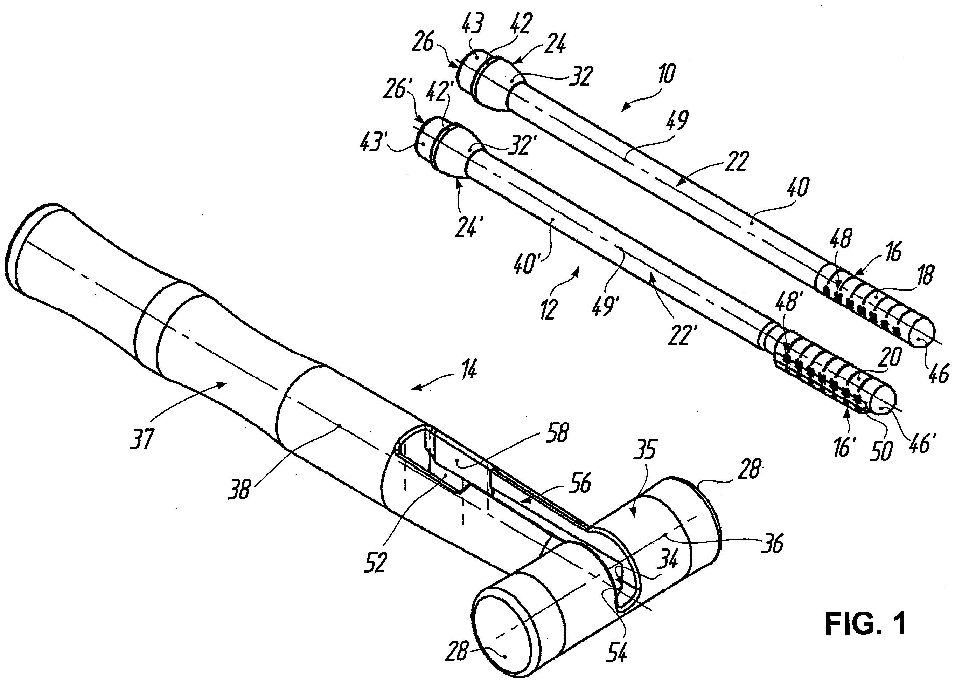

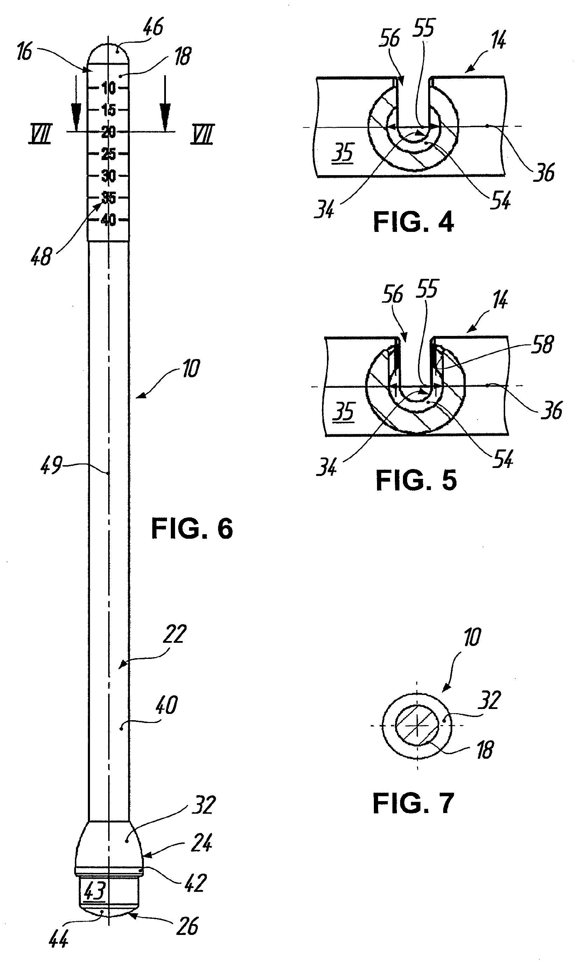

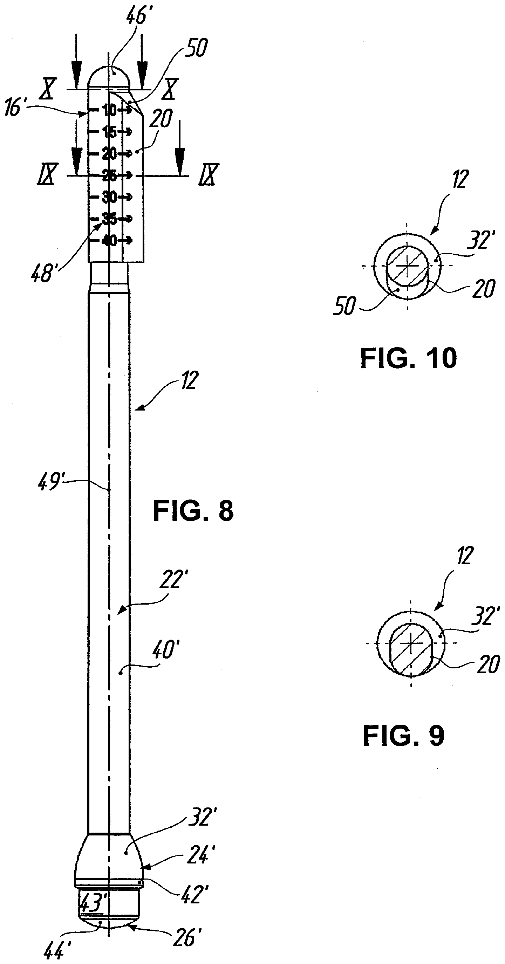

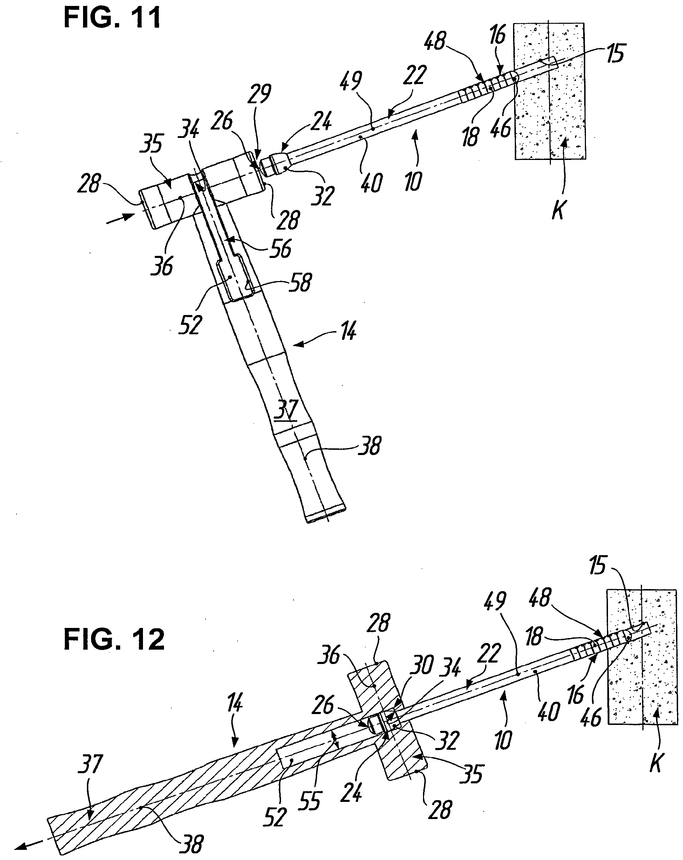

Die Fig. 1 zeigt exemplarisch einen chirurgischen Instrumentensatz mit drei Werkzeugen, nämlich zwei stabförmigen Treibwerkzeugen 10, 12 und einer hammerförmigen Impulsmasse 14, mittels der auf noch zu beschreibende Weise äußere Kräfte an den Treibwerkzeugen 10, 12 aufgebracht werden können. Bei den Treibwerkzeugen 10, 12 handelt es sich im dargestellten Ausführungsbeispiel um Dilatatoren zum Aufweiten von Knochenbohrungen 15, 15' (vgl. die Fig. 11 bis 14) in einem (dort schematisch angedeuteten) Knochen K, wozu das eine Treibwerkzeug 10 an seinem ersten Ende 16 einen für eine symmetrische Aufweitung der Knochenbohrung 15 ausgestalteten Aufweitungsabschnitt 18 aufweist, wie nachfolgend anhand der Fig. 6, 7, 11 und 12 noch näher beschrieben wird, während das andere Treibwerkzeug 12 an seinem ersten Ende 16' einen für eine asymmetrische Aufweitung der Knochenbohrung 15' ausgebildeten Aufweitungsabschnitt 20 besitzt, wie im Folgenden unter Bezugnahme auf die Fig. 8 bis 10, 13 und 14 ebenfalls noch im Detail erläutert wird. Den jeweiligen Einsatzerfordernissen entsprechend kann der chirurgische Instrumentensatz natürlich auch nur ein oder deutlich mehr verschiedene Treibwerkzeuge besitzen, was für das Verständnis der vorliegenden Erfindung aber nicht erheblich ist, so dass der Einfachheit halber - beispielhaft und stellvertretend für ggf. weitere Treibwerkzeuge - in den Figuren nur zwei verschiedene Treibwerkzeuge dargestellt sind.

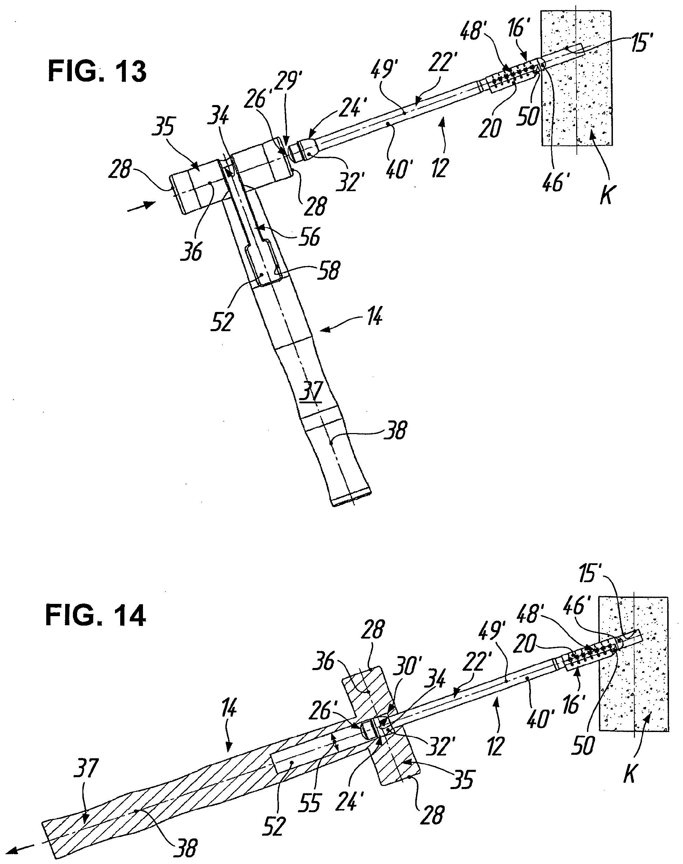

Jedes Treibwerkzeug 10, 12 besitzt einen Schaft 22, 22' und ein zweites Ende 24, 24', welches jeweils zur Einleitung der äußeren Kräfte in Richtung des ersten Endes 16, 16' oder davon weg ausgebildet ist. Wesentlich hierbei ist, dass das jeweilige zweite Ende 24, 24' des Treibwerkzeugs 10 bzw. 12 und die Impulsmasse 14 jeweils (wenigstens) eine Kraftübertragungsfläche 26, 26', 28 besitzen, die zusammen eine Flächenpaarung 29, 29' (siehe die Fig. 11 und 13) bilden, über die die äußeren Kräfte in Richtung des ersten Endes 16, 16' eingeleitet werden können, und darüber hinaus auch mit komplementären Strukturen 30, 30' (vgl. die Fig. 12 und 14) versehen sind, über welche die äußeren Kräfte in Richtung weg vom ersten Ende 16, 16' aufgebracht werden können. Mit anderen Worten gesagt ist ein und dieselbe Impulsmasse 14 so ausgebildet, dass mit ihr die Treibwerkzeuge 10 bzw. 12 in die Knochenbohrung 15, 15' eingetrieben und anschließend wieder aus der jeweiligen Knochenbohrung 15, 15' ausgetrieben werden können, ohne dass hierzu das Schlagwerkzeug gewechselt werden müsste. Im gezeigten Ausführungsbeispiel weisen die vorerwähnten komplementären Strukturen 30, 30' hierbei eine dem ersten Ende 16, 16' zugewandte Einleitungsfläche 32, 32' am zweiten Ende 24, 24' des Schafts 22, 22' des Treibwerkzeugs 10 bzw. 12 und eine damit zur Krafteinleitung schlagend in Anlage bringbare Gegenfläche 34 an der Impulsmasse 14 auf.

Wie insbesondere in den Fig. 1 bis 3 weiterhin zu erkennen ist besitzt die Impulsmasse 14 einen Hammerkopf 35, der auf voneinander abgewandten Seiten jeweils eine ebene Schlagfläche als mögliche Kraftübertragungsfläche 28 - also zwei Kraftübertragungsflächen 28 - aufweist und dessen Mittelachse eine erste Achse 36 der Impulsmasse 14 definiert, und einen Hammerstiel 37, dessen Mittelachse eine zweite Achse 38 der Impulsmasse 14 definiert. Dabei ist die jeweilige Kraftübertragungsfläche 28 für die äußeren Kräfte in Richtung des ersten Endes 16, 16' senkrecht zur ersten Achse 36 ausgerichtet, während die Gegenfläche 34 für die äußeren Kräfte in Richtung weg vom ersten Ende 16, 16' senkrecht zur zweiten Achse 38 ausgerichtet ist. Im Ergebnis sind die jeweilige Kraftübertragungsfläche 28 der Flächenpaarung 29, 29' zur Einleitung der äußeren Kräfte in Richtung des ersten Endes 16, 16' des Schafts 22, 22' des Treibwerkzeugs 10, 12 und die Gegenfläche 34 der komplementären Strukturen 30, 30' zur Aufbringung der äußeren Kräfte in Richtung weg vom ersten Ende 16, 16' an der Impulsmasse 14 also senkrecht zu verschiedenen Achsen 36, 38 der Impulsmasse 14 ausgerichtet. Somit kann der Operateur die Impulsmasse 14 zum Einschlagen des jeweiligen Treibwerkzeugs 10, 12 in gewohnter Weise wie einen Hammer benutzen, wobei er hier aufgrund der bilateralen Symmetrie des Hammerkopfs 35 die Wahl zwischen zwei Kraftübertragungsflächen 28 hat; beim Austreiben des jeweiligen Treibwerkzeugs 10, 12 indes hilft der Hammerstiel 37 bei der Orientierung der Impulsmasse 14 bezüglich der gebotenen Austreibrichtung, wobei "im Schlag" ein leichtes Öffnen der Hammerführungshand zur tunlichst ungebremsten Übertragung des Austreibimpulses auf das Treibwerkzeug 10, 12 ohne Kontrollverlust möglich ist.

Weitere Details zu den Treibwerkzeugen 10, 12 sind insbesondere den Fig. 6 bis 10 zu entnehmen. Demgemäß ist das zweite Ende 24, 24' des Schafts 22, 22' zur Einleitung der axialen Austreibkräfte mit einer Querschnittserweiterung versehen, an der die dem ersten Ende 16, 16' zugewandte Einleitungsfläche 32, 32' ausgebildet ist, so dass Letztere gegenüber einem sich zwischen den Enden 16, 16' und 24, 24' des Schafts 22, 22' erstreckenden Mittelabschnitt 40, 40' des Schafts 22, 22' allseits radial übersteht, mithin eine Ringfläche bildet, wie in den Ansichten gemäß den Fig. 7, 9 und 10 besonders gut zu erkennen ist. Diese ringförmige Einleitungsfläche 32, 32' ist gemäß den Fig. 6 und 8 ballig ausgestaltet, wölbt sich also nach radial außen vor.

An die ballig geformte Einleitungsfläche 32, 32' schließt sich stufenlos ein kurzer zylindrischer Abschnitt 42, 42' und hieran über eine Stufe ein weiterer zylindrischer Abschnitt 43, 43' kleineren Durchmessers an, bevor das zweite Ende 24, 24' des Schafts 22, 22' mit einem kugelkalottenförmigen Endabschnitt 44, 44' abschließt, der die Kraftübertragungsfläche 26, 26' zur Einleitung der äußeren Kräfte in Richtung des ersten Endes 16, 16' des Schafts 22, 22' ausbildet.

Am anderen, ersten Ende 16, 16' des Schafts 22, 22' schließt das Treibwerkzeug 10, 12 mit einem weiteren kugelkalottenförmigen, genauer halbkugelförmigen Endabschnitt 46, 46' ab, dessen Radius hier im Wesentlichen dem Radius des Schafts 22, 22' entspricht. Ein solcher Endabschnitt 46, 46' erleichtert in vorteilhafter Weise insbesondere ein bezüglich der Knochenbohrung 15, 15' zentriertes Ausrichten des jeweiligen Treibwerkzeugs 10, 12.

Gemäß Fig. 6 folgt am ersten Ende 16 des Treibwerkzeugs 10 dem halbkugelförmigen Endabschnitt 46 axial unmittelbar und radial stufenlos der durchmessergleiche Aufweitungsabschnitt 18, der bei dieser Werkzeugvariante für eine symmetrische Dilatation der Knochenbohrung 15 einen kreisförmigen Querschnitt mit einem vorbestimmten Durchmesser aufweist, wie am besten in Fig. 7 zu erkennen ist. Aufgrund der hiermit bewirkten symmetrischen Aufweitung der Knochenbohrung 15, bei der das angrenzende Knochenmaterial über dem Umfang des Aufweitungsabschnitts 18 gesehen gleichmäßig radial nach außen verdrängt wird, ist diese Art der Aufweitung besonders schonend für den Knochen K. Um ein Maß für die Tiefe der aufgeweiteten Knochenbohrung 15 zu erhalten, kann das Treibwerkzeug 10 im Bereich des Aufweitungsabschnitts 18 mit einer ggf. beschrifteten Tiefen- bzw. Längenskala 48 versehen sein.

Weitere Details zu dem Treibwerkzeug 12 zur asymmetrischen Dilatation sind den Fig. 8 bis 10 zu entnehmen. Gemäß den Fig. 8 und 9 ist die Querschnittform des Aufweitungsabschnitts 20 am ersten Ende 16' des Schafts 22' für die asymmetrische Dilatation ausgehend von einem bezüglich einer Mittelachse 49' des Schafts 22' zentrierten, kreisförmigen Grundquerschnitt auf einer Seite, d.h. in Fig. 8 nach rechts (bzw. Fig. 9 nach unten) erhaben ausgebildet. Genauer gesagt besitzt der Aufweitungsabschnitt 20 im dargestellten Ausführungsbeispiel einen ovalen Querschnitt, dessen Umfangskontur durch zwei Halbkreise gleichen Durchmessers und zwei die Halbkreise verbindenden Geraden gleicher Länge gebildet ist, wobei der Mittelpunkt einer der Halbkreise auf der Mittelachse 49' sitzt. Somit wird die Knochenbohrung 15' beim Einschlagen des Treibwerkzeugs 12 im Querschnitt gesehen asymmetrisch ausgeformt, um z.B. bei der eingangs beschriebenen autologen Wiederherstellung des vorderen Kreuzbandes des Kniegelenks nach der sogenannten "All-Press-Fit"-Technik zu einer oder beiden Seiten der Knochenbohrung 15' ein Sehnenlager auszubilden, in welches ein nur begrenzt verformbares Sehnenimplantat ausweichen kann, wenn ein Knochenimplantat zur Verankerung eingeschlagen wird. Hierdurch wird insbesondere ein übermäßiges Verquetschen eines im Verhältnis querschnittsgroßen Sehnenimplantats verhindert; auch wird auf diese Weise - verglichen mit symmetrisch ausgebildeten "Press-Fit"-Verbindungen - bei querschnittsgrößeren Sehnenimplantaten die Möglichkeit der Bildung von den Einheilprozess verschlechternden Spalten oder Hohlräumen weiter reduziert.

Wie ferner die Fig. 8 und 10 zeigen erstreckt sich zwischen dem halbkugelförmigen Endabschnitt 46' und dem Aufweitungsabschnitt 20 des Treibwerkzeugs 12 zur asymmetrischen Dilatation ein Übergangsabschnitt 50, dessen Querschnitt sich von einem größten Querschnitt des Endabschnitts 46' zu dem Querschnitt des Aufweitungsabschnitts 20 kontinuierlich erweitert, was wiederum einem knochenmaterialschonenden Aufweiten der Knochenbohrung 15' förderlich ist. Über eine geeignete Wahl der Form der Querschnittserweiterung des Übergangsabschnitts 50 kann hierbei auf den Fortschritt der Aufweitung und die Höhe der örtlichen Aufweitungskräfte in radialer und axialer Richtung in Abhängigkeit vom Werkzeugvortrieb gezielt Einfluss genommen werden.

Bezüglich der Treibwerkzeuge 10, 12 ist abschließend noch anzumerken, dass deren Aufweitungsabschnitte 18 bzw. 20 zur Ausbildung von Sehnenimplantatlagern nach der vorbeschriebenen "All-Press-Fit"-Technik in der rekonstruktiven Chirurgie exakt bemessene Querschnittsformen und -flächen aufweisen können, wie es in der zeitgleicheingereichten deutschen Patentanmeldung DE 10 2015 007 541.1 derselben Anmelder im Detail beschrieben wird, auf die hiermit bezüglich der Bemessungsregeln ausdrücklich verwiesen wird.

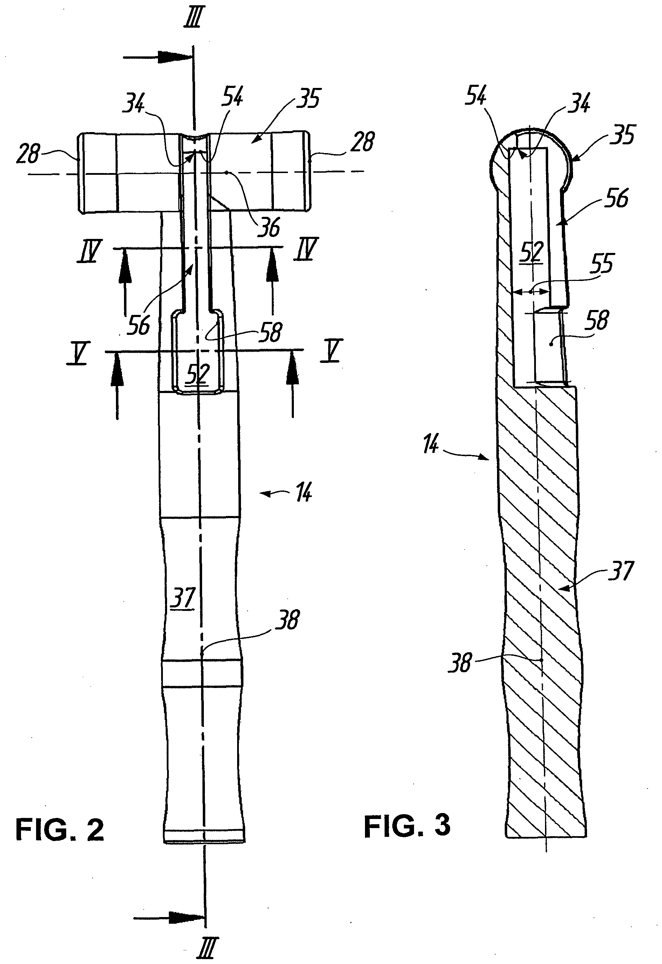

Die Fig. 2 bis 5 zeigen weitere Einzelheiten der hammerförmigen Impulsmasse 14. Wie insbesondere den Fig. 1 bis 3 zu entnehmen ist besitzt die Impulsmasse 14 zur Aufnahme des Mittelabschnitts 40, 40' des Schafts 22, 22' des jeweiligen Treibwerkzeugs 10, 12 eine Aussparung 52, die eine die Gegenfläche 34 an der Impulsmasse 14 ausbildende Schulter 54 aufweist, die an der dem ersten Ende 16, 16' zugewandten, radial überstehenden Einleitungsfläche 32, 32' am zweiten Ende 24, 24' des Schafts 22, 22' schlagend in Anlage gebracht werden kann. Gemäß den Fig. 2 und 3 ist diese Aussparung 52 mit der zweiten Achse 38 der Impulsmasse 14 ausgefluchtet und erstreckt sich vom Hammerstiel 37 in den Hammerkopf 35 hinein, wo die Aussparung 52 mit ihrer Schulter 54 in den Fig. 2 und 3 nach oben offen endet. Demnach ist die Gegenfläche 34 an der Schulter 54 auf einer bezüglich der Aussparung 52 inneren Seite der Schulter 54 ausgebildet, wobei die Aussparung 52 vor der Schulter 54 einen lichten Querschnitt 55 aufweist (siehe die Fig. 3 bis 5), der für ein ungehindertes Bewegen auch des zweiten, verdickten Endes 24, 24' des Schafts 22, 22' des jeweiligen Treibwerkzeugs 10, 12 in der Aussparung 52 größer als der größte Querschnitt des zweiten Endes 24, 24' ist.

Gemäß den Fig. 1 bis 3 ist die Impulsmasse 14 weiterhin mit einem Schlitz 56 versehen, der - sich ebenfalls von dem Hammerstiel 37 in den Hammerkopf 35 hinein erstreckend - parallel zu der Aussparung 52 verläuft und über den der Mittelabschnitt 40, 40' des Schafts 22, 22' des jeweiligen Treibwerkzeugs 10, 12 in die Aussparung 52 eingeführt werden kann. Schließlich weist der Hammerstiel 37 eine sich in Richtung quer zur zweiten Achse 38 erstreckende und an den Schlitz 56 anschließende Fügeöffnung 58 auf, die in der Aussparung 52 endet. Die in der Draufsicht gemäß Fig. 2 gesehen im Wesentlichen rechteckige Fügeöffnung 58 dient dazu, das verdickte, zweite Ende 24, 24' des Schafts 22, 22' des jeweiligen Treibwerkzeugs 10, 12 quer zum Hammerstiel 37 in die Aussparung 52 einzuführen, bevor das Treibwerkzeug 10, 12 mittels der Impulsmasse 14 aus der jeweiligen Knochenbohrung 15 bzw. 15' ausgetrieben wird.

Die Fig. 11 bis 14 zeigen den vorbeschriebenen chirurgischen Instrumentensatz im Einsatz, wobei in den Fig. 11 und 12 die Knochenbohrung 15 im Knochen K mittels des Treibwerkzeugs 10 symmetrisch aufgeweitet wird, während in den Fig. 13 und 14 die Knochenbohrung 15' im Knochen K mittels des Treibwerkzeugs 12 asymmetrisch aufgeweitet wird. Die Art und Weise der Verwendung der hammerförmigen Impulsmasse 14 ist dabei die gleiche, so dass nachfolgend das Vorgehen für einen symmetrischen Dilatationsschritt (Fig. 11 und 12) und das Vorgehen für einen asymmetrischen Dilatationsschritt (Fig. 13 und 14) zugleich beschrieben werden.

Ist der Operateur ein Rechtshänder, so hält er das Treibwerkzeug 10, 12 am Mittelabschnitt 40, 40' des Schafts 22, 22' nahe dem zweiten Ende 24, 24' in der linken Hand und setzt das Treibwerkzeug 10, 12 mit im Verhältnis geringer Kraft mit dem halbkugelförmigen Endabschnitt 46, 46' an der vorher schneidend bzw. spanend erzeugten Knochenbohrung 15, 15' an. Dabei zentriert sich das Treibwerkzeug 10, 12 infolge der runden Form des Endabschnitts 46, 46' eingangs der Knochenbohrung 15, 15' selbsttätig.

In der rechten Hand hält der Operateur die Impulsmasse 14 am Hammerstiel 37, so dass er durch schwingende bzw. bogenförmige Bewegung der Impulsmasse 14 das Treibwerkzeug 10, 12 wie einen Nagel in die Knochenbohrung 15, 15' mit einer Mehrzahl von Schlägen eintreiben bzw. einschlagen kann. Dabei trifft die Impulsmasse 14 mit einer vom Operateur vorgewählten Kraftübertragungsfläche 28 des Hammerkopfs 35 schlagend auf der Kraftübertragungsfläche 26, 26' am Treibwerkzeug 10, 12 auf, wie in den Fig. 11 und 13 gezeigt. Infolgedessen überträgt sich der Impuls der bewegten Impulsmasse 14 über die Flächenpaarung 29, 29' auf das Treibwerkzeug 10, 12 in Richtung des Pfeils in den Fig. 11 und 13, wodurch das Treibwerkzeug 10, 12 mit seinem ersten Ende 16, 16' in die Knochenbohrung 15, 15' eingetrieben wird.

Da der Querschnitt des Aufweitungsabschnitts 18, 20 am ersten Ende 16, 16' des Treibwerkzeugs 10, 12 größer ist als der ursprüngliche Querschnitt der Knochenbohrung 15, 15', wird Letztere definiert aufgeweitet. Zur Führung hält der Operateur dabei das Treibwerkzeug 10, 12 weiterhin mit seiner linken Hand am Mittelabschnitt 40, 40' des Schafts 22, 22'. Mittels der Längenskala 48, 48' am Aufweitungsabschnitt 18, 20 kann der Operateur kontrollieren, wie weit er das Treibwerkzeug 10, 12 in die Knochenbohrung 15, 15' eintreibt.

Ist die gewünschte Eintreibtiefe erreicht, setzt der Operateur die Impulsmasse 14 - ohne diese vorher aus der rechten Hand zu legen - auf das Treibwerkzeug 10, 12, um dieses aus der Knochenbohrung 15, 15' auszutreiben. Mit der linken Hand führt er dabei das Treibwerkzeug 10, 12 an dessen Schaft 22, 22'. Genauer gesagt richtet der Operateur den Hammerstiel 37 bezüglich der Mittelachse 49 des Treibwerkzeugs 10, 12 aus, wobei der Hammerkopf 35 in Richtung der Knochenbohrung 15, 15' zeigt. Nun legt der Operateur die Impulsmasse 14 über das Treibwerkzeug 10, 12, wobei das verdickte zweite Ende 24, 24' des Treibwerkzeugs 10, 12 über die Fügeöffnung 58 und der Mittelabschnitt 40, 40' des Treibwerkzeugs 10, 12 über den Schlitz 56 in die Aussparung 52 in der Impulsmasse 14 gelangen. Jetzt kann der Operateur mit seiner rechten Hand die Impulsmasse 14 über den Hammerstiel 37 von der Knochenbohrung 15, 15' weg beschleunigen. Die Impulsmasse 14 bewegt sich dabei über das in der Aussparung 52 aufgenommene, verdickte zweite Ende 24, 24' des Treibwerkzeugs 10, 12 weg, wobei das zweite Ende 24, 24' des vom Operateur mit links gehaltenen Treibwerkzeugs 10, 12 die Impulsmasse 14 auch führt. Da der lichte Querschnitt 55 der Aussparung 52 größer ist als der größte Querschnitt des zweiten Endes 24, 24' des Treibwerkzeugs 10, 12, wird diese Längsbewegung der Impulsmasse 14 nicht behindert. Die Breite des Schlitzes 56 in der Impulsmasse 14 ist indes kleiner als der größte Querschnitt des zweiten Endes 24, 24' des Treibwerkzeugs 10, 12, so dass das zweite Ende 24, 24' des Treibwerkzeugs 10, 12 ein "Abheben" der beschleunigten Impulsmasse 14 vom Treibwerkzeug 10, 12 verhindert.

Infolge dieser relativen Längsbewegung trifft die Impulsmasse 14 mit ihrer an der Schulter 54 im Hammerkopf 35 ausgebildeten Gegenfläche 34 an der balligen Einleitungsfläche 32, 32' am zweiten Ende 24, 24' des Treibwerkzeugs 10, 12 auf, wie in den Fig. 12 und 14 gezeigt. Folglich überträgt sich der Impuls der bewegten Impulsmasse 14 über die komplementären Strukturen 30, 30' auf das Treibwerkzeug 10, 12 in Richtung des Pfeils in den Fig. 12 und 14, wodurch das Treibwerkzeug 10, 12 aus der Knochenbohrung 15, 15' - ggf. Schritt für Schritt - ausgetrieben wird. Kurz bevor die komplementären Strukturen 30, 30' (jeweils) impulsübertragend zur Anlage gelangen kann der Operateur die rechte Hand leicht öffnen, so dass die Impulsmasse 14 vom Operateur im Wesentlichen kraftfrei am Hammerstiel 37 geführt wird, im Moment der Impulsübertragung aber keine unkontrollierten Querkräfte von der Impulsmasse 14 in das Treibwerkzeug 10, 12 gelangen. Auch das Austreiben des Treibwerkzeugs 10, 12 aus der Knochenbohrung 15, 15' erfolgt - wie das Eintreiben - in der Regel mit mehreren Bewegungen der Impulsmasse 14,.wie schon angedeutet.

Aus der obigen Beschreibung ist ersichtlich, dass der Operateur die Treibwerkzeuge 10, 12 mit ein und derselben Impulsmasse 14 ein- und austreiben kann, ohne dass er hierzu die jeweiligen Werkzeuge aus der Hand legen oder wechseln müsste. Ist der Operateur ein Linkshänder, so würde er die Impulsmasse 14 in analoger Weise mit links führen und das jeweilige Treibwerkzeug 10, 12 mit rechts halten.

Ein chirurgischer Instrumentensatz umfasst mindestens ein stabförmiges Treibwerkzeug, das einen Schaft mit einem ersten Ende und einem zweiten Ende aufweist, welches zur Einleitung von äußeren Kräften in Richtung des ersten Endes oder davon weg ausgebildet ist, und eine Impulsmasse zum Aufbringen von äußeren Kräften am zweiten Ende. Dabei besitzen das zweite Ende des Treibwerkzeugs und die Impulsmasse jeweils eine Kraftübertragungsfläche, die zusammen eine Flächenpaarung bilden, über die die äußeren Kräfte in Richtung des ersten Endes des Treibwerkzeugs eingeleitet werden können. Darüber hinaus sind das Treibwerkzeug und die Impulsmasse mit komplementären Strukturen versehen, über welche die äußeren Kräfte in Richtung weg vom ersten Ende des Treibwerkzeugs aufgebracht werden können, so dass bei einer Operation die Impulsmasse zum Ein- bzw. Austreiben des Treibwerkzeugs nicht gewechselt werden muss.

### BEZUGSZEICHENLISTE

- 10: Treibwerkzeug
- 12: Treibwerkzeug
- 14: Impulsmasse
- 15, 15': Knochenbohrung
- 16, 16': erstes Ende
- 18: Aufweitungsabschnitt
- 20: Aufweitungsabschnitt
- 22, 22': Schaft
- 24, 24': zweites Ende
- 26, 26': Kraftübertragungsfläche
- 28: Kraftübertragungsfläche
- 29, 29': Flächenpaarung
- 30, 30': komplementäre Strukturen
- 32, 32': Einleitungsfläche
- 34: Gegenfläche
- 35: Hammerkopf
- 36: erste Achse
- 37: Hammerstiel
- 38: zweite Achse
- 40, 40': Mittelabschnitt
- 42, 42': zylindrischer Abschnitt
- 43, 43': zylindrischer Abschnitt
- 44, 44': kugelkalottenförmiger Endabschnitt
- 46, 46': halbkugelförmiger Endabschnitt
- 48, 48': Längenskala
- 49, 49': Mittelachse
- 50: Übergangsabschnitt
- 52: Aussparung
- 54: Schulter
- 55: lichter Querschnitt
- 56: Schlitz
- 58: Fügeöffnung
- K: Knochen

## Patentansprüche

1. Chirurgischer Instrumentensatz, mit
mindestens einem stabförmigen Treibwerkzeug (10, 12), das einen Schaft (22, 22') mit einem ersten Ende (16, 16') und einem zweiten Ende (24, 24') aufweist, welches zur Einleitung von äußeren Kräften in Richtung des ersten Endes (16, 16') oder davon weg ausgebildet ist, und
einer Impulsmasse (14) zum Aufbringen der äußeren Kräfte am zweiten Ende (24, 24'),
wobei das zweite Ende (24, 24') und die Impulsmasse (14) jeweils eine Kraftübertragungsfläche (26, 26', 28) besitzen, die zusammen eine Flächenpaarung (29, 29') bilden, über die die äußeren Kräfte in Richtung des ersten Endes (16, 16') einleitbar sind, sowie mit komplementären Strukturen (30, 30') versehen sind, über welche die äußeren Kräfte in Richtung weg vom ersten Ende (16, 16') aufbringbar sind,
wobei die komplementären Strukturen (30, 30') eine dem ersten Ende (16, 16') zugewandte Einleitungsfläche (32, 32') am zweiten Ende (24, 24') des Schafts (22, 22') des Treibwerkzeugs (10, 12) und eine damit zur Krafteinleitung schlagend in Anlage bringbare Gegenfläche (34) an der Impulsmasse (14) aufweisen,
wobei die Impulsmasse (14) die Form eines Hammers aufweist, mit einem Hammerkopf (35), dessen Mittelachse eine erste Achse (36) der Impulsmasse (14) definiert, und einem Hammerstiel (37), dessen Mittelachse eine von der ersten Achse (36) verschiedene zweite Achse (38) der Impulsmasse (14) definiert, und
wobei die Kraftübertragungsfläche (28) für die äußeren Kräfte in Richtung des ersten Endes (16, 16') senkrecht zur ersten Achse (36) ausgerichtet ist,
**dadurch gekennzeichnet, dass** die Gegenfläche (34) für die äußeren Kräfte in Richtung weg vom ersten Ende (16, 16') senkrecht zur zweiten Achse (38) ausgerichtet ist.

2. Instrumentensatz nach Anspruch. 1, **dadurch gekennzeichnet, dass** die dem ersten Ende (16, 16') zugewandte Einleitungsfläche (32, 32') am zweiten Ende (24, 24') des Schafts (22, 22') gegenüber einem sich zwischen den Enden (16, 24; 16', 24') des Schafts (22, 22') erstreckenden Mittelabschnitt (40, 40') des Schafts (22, 22') radial übersteht.

3. Instrumentensatz nach Anspruch 2, **dadurch gekennzeichnet, dass** die dem ersten Ende (16, 16') zugewandte Einleitungsfläche (32, 32') am zweiten Ende (24, 24') des Schafts (22, 22') eine Ringfläche ist.

4. Instrumentensatz nach einem der vorhergehenden Ansprüche, **dadurch gekennzeichnet, dass** die dem ersten Ende (16, 16') zugewandte Einleitungsfläche (32, 32') am zweiten Ende (24, 24') des Schafts (22, 22') ballig ausgestaltet ist.

5. Instrumentensatz nach einem der vorhergehenden Ansprüche, **dadurch gekennzeichnet, dass** das zweite Ende (24, 24') des Schafts (22, 22') einen kugelkalottenförmigen Endabschnitt (44, 44') aufweist, der die Kraftübertragungsfläche (26, 26') zur Einleitung der äußeren Kräfte in Richtung des ersten Endes (16, 16') des Schafts (22, 22') ausbildet.

6. Instrumentensatz nach wenigstens dem Anspruch 2, **dadurch gekennzeichnet, dass** die Impulsmasse (14) zur Aufnahme des Mittelabschnitts (40, 40') des Schafts (22, 22') eine Aussparung (52) besitzt, die eine die Gegenfläche (34) an der Impulsmasse (14) ausbildende Schulter (54) aufweist, an der die dem ersten Ende (16, 16') zugewandte, radial überstehende Einleitungsfläche (32, 32') am zweiten Ende (24, 24') des Schafts (22, 22') schlagend in Anlage bringbar ist.

7. Instrumentensatz nach Anspruch 6, **dadurch gekennzeichnet, dass** die Impulsmasse (14) mit einem Schlitz (56) versehen ist, der parallel zu der Aussparung (52) verläuft und über den der Mittelabschnitt (40, 40') des Schafts (22, 22') in die Aussparung (52) einführbar ist.

8. Instrumentensatz nach Anspruch 6 oder 7, **dadurch gekennzeichnet, dass** die Gegenfläche (34) an der Schulter (54) auf einer bezüglich der Aussparung (52) inneren Seite der Schulter (54) ausgebildet ist und die Aussparung (52) vor der Schulter (54) einen lichten Querschnitt (55) aufweist, der für ein ungehindertes Bewegen auch des zweiten Endes (24, 24') des Schafts (22, 22') in der Aussparung (52) größer ist als der größte Querschnitt des zweiten Endes (24, 24').

9. Instrumentensatz nach wenigstens dem Anspruch 6, **dadurch gekennzeichnet, dass** die Aussparung (52) zur Aufnahme des Schafts (22, 22') des Treibwerkzeugs (10, 12) mit der zweiten Achse (38) der Impulsmasse (14) ausgefluchtet ist und sich vom Hammerstiel (37) in den Hammerkopf (35) hinein erstreckt, wo die Aussparung (52) mit ihrer Schulter (54) offen endet.

10. Instrumentensatz nach wenigstens den Ansprüchen 7 und 9, **dadurch gekennzeichnet, dass** der Hammerstiel (37) eine sich in Richtung quer zur zweiten Achse (38) erstreckende und an den Schlitz (56) anschließende Fügeöffnung (58) aufweist, die in der Aussparung (52) endet und dazu dient, das zweite Ende (24, 24') des Schafts (22, 22') in die Aussparung (52) einzuführen.

11. Instrumentensatz nach einem der vorhergehenden Ansprüche, **dadurch gekennzeichnet, dass** der Hammerkopf (35) auf voneinander abgewandten Seiten jeweils eine ebene Schlagfläche als mögliche Kraftübertragungsfläche (28) aufweist.

12. Instrumentensatz nach einem der vorhergehenden Ansprüche, **dadurch gekennzeichnet, dass** das Treibwerkzeug (10, 12) ein Dilatator zum Aufweiten einer Knochenbohrung (15, 15') ist, der an seinem ersten Ende (16, 16') einen für eine symmetrische oder asymmetrische Aufweitung der Knochenbohrung ausgestalteten Aufweitungsabschnitt (18, 20) aufweist.

## Claims

1. Surgical instrument kit comprising
at least one rod-shaped drive tool (10, 12) which comprises a shank (22, 22') with a first end (16, 16') and a second end (24, 24'), which is configured for introduction of external forces in the direction of the first end (16, 16') or away therefrom, and
an impulse mass (14) for application of the external forces to the second end (24, 24'),
wherein the second end (24, 24') and the impulse mass (14) each have a force transmission surface (26, 26', 28) which together form a surface pairing (29, 29'), by way of which the external forces are introducible in the direction of the first end (16, 16'), and are provided with complementary structures (30, 30') by way of which the external forces can be applied in a direction away from the first end (16, 16'),
wherein the complementary structures (30, 30') have an introduction surface (32, 32'), which faces the first end (16, 16'), at the second end (24, 24') of the shank (22, 22') of the drive tool (10, 12) and a counter-surface (34), which can be brought into impacting contact therewith for force introduction, at the impulse mass (14),
wherein the impulse mass (14) has the form of a hammer with a hammer head (35), the center axis of which defines a first axis (36) of the impulse mass (14), and a hammer post (37), the center axis of which defines a second axis (38), which is different from the first axis (36), of the impulse mass (14), and
wherein the force transmission surface (28) for the external forces is oriented in the direction of the first end (16, 16') perpendicularly to the first axis (36),
**characterized in that** the counter-surface (34) for the external forces is oriented in direction away from the first end (16, 16') perpendicularly to the second axis (38).

2. Instrument kit according to claim 1, **characterized in that** the introduction surface (32, 32'), which faces the first end (16, 16'), at the second end (24, 24') of the shank (22, 22'), projects radially relative to a center section (40, 40') of the shank (22, 22'), which extends between the ends (16, 24; 16', 24') of the shank (22, 22').

3. Instrument kit according to claim 2, **characterized in that** the introduction surface (32, 32'), which faces the first end (16, 16'), at the second end (24, 24') of the shank (22, 22') is an annular surface.

4. Instrument kit according to any one of the preceding claims, **characterized in that** the introduction surface (32, 32'), which faces the first end (16, 16'), at the second end (24, 24') of the shank (22, 22') is of crowned shape.

5. Instrument kit according to any one of the preceding claims, **characterized in that** the second end (24, 24') of the shank (22, 22') has a spherically cap-shaped end section (44, 44') which forms the force transmission surface (26, 26') for introduction of the external forces in the direction of the first end (16, 16') of the shank (22, 22').

6. Instrument kit according to at least claim 2, **characterized in that** the impulse mass (14) has a cut-out (52) for reception of the center section (40, 40') of the shank (22, 22'), the cut-out having a shoulder (54) which forms the counter-surface (34) at the impulse mass (14) and with which the radially projecting introduction surface (32, 32'), which faces the first end (16, 16'), at the second end (24, 24') of the shank (22, 22') can be brought into impacting contact.

7. Instrument kit according to claim 6, **characterized in that** the impulse mass (14) is provided with a slot (56) which extends parallel to the cut-out (52) and by way of which the center section (40, 40') of the shank (22, 22') is introducible into the cut-out (52).

8. Instrument kit according to claim 6 or 7, **characterized in that** the counter-surface (34) is formed at the shoulder (54) on a side of the shoulder (54) which is inner with respect to the cut-out (52) and the cut-out (52) has in front of the shoulder (54) a clear cross-section (55) which for unhindered movement also of the second end (24, 24') of the shank (22, 22') in the cut-out (52) is greater than the largest cross-section of the second end (24, 24').

9. Instrument kit according to at least claim 6, **characterized in that** the cut-out (52) for reception of the shank (22, 22') of the drive tool (10, 12) is aligned with the second axis (38) of the impulse mass (14) and extends from the hammer post (37) into the hammer head (35), where the cut-out (52) ends by its shoulder (54) to be open.

10. Instrument set according to at least claims 7 and 9, **characterized in that** the hammer post (37) has a joining opening (58), which extends in a direction transverse to the second axis (38) and is connected with the slot (56) and which ends in the cut-out (52) and serves the purpose of introducing the second end (24, 24') of the shank (22, 22') into the cut-out (52).

11. Instrument kit according to any one of the preceding claims, **characterized in that** the hammer head (35) has on mutually remote sides a respective planar impact surface as a possible force transmission surface (28).

12. Instrument kit according to any one of the preceding claims, **characterized in that** the drive tool (10, 12) is a dilator for expansion of a bone bore (15, 15'), which has at its first end (16, 16') an expansion section (18, 20) designed for a symmetrical or an asymmetrical widening of the bone bore.

## Revendications

1. Ensemble d'instruments chirurgicaux, avec
au moins un outil d'entraînement (10, 12) en forme de barre qui présente une tige (22, 22') avec une première extrémité (16, 16') et une deuxième extrémité (24, 24'), qui est agencée pour introduire des forces externes en direction de la première extrémité (16, 16') ou dans la direction opposée à celle-ci, et
une masse d'impulsion (14) pour appliquer les forces externes à la deuxième extrémité (24, 24'),
où la deuxième extrémité (24, 24') et la masse d'impulsion (14) possèdent chacune une surface de transmission de force (26, 26', 28), qui forment ensemble une paire de surfaces (29, 29') par l'intermédiaire de laquelle les forces externes peuvent être introduites en direction de la première extrémité (16, 16'), et sont munies de structures complémentaires (30, 30') par l'intermédiaire desquelles les forces externes peuvent être appliquées dans la direction opposée à la première extrémité (16, 16'),
où les structures complémentaires (30, 30') présentent une surface d'introduction (32, 32') tournée vers la première extrémité (16, 16') au niveau de la deuxième extrémité (24, 24') de la tige (22, 22') de l'outil d'entraînement (10, 12) et une contre-surface (34) au niveau de la masse d'impulsion (14) qui peut être amenée en butée avec la précédente par frappe pour l'introduction des forces,
où la masse d'impulsion (14) présente la forme d'un marteau, avec une tête de marteau (35) dont l'axe central définit un premier axe (36) de la masse d'impulsion (14), et un manche de marteau (37) dont l'axe central définit un deuxième axe (38) de la masse d'impulsion (14) différent du premier axe (36), et
où la surface de transmission de force (28) pour les forces externes en direction de la première extrémité (16, 16') est orientée perpendiculairement au premier axe (36),
**caractérisé en ce que** la contre-surface (34) pour les forces externes dans la direction opposée à la première extrémité (16, 16') est orientée perpendiculairement au deuxième axe (38).

2. Ensemble d'instruments selon la revendication 1, **caractérisé en ce que** la surface d'introduction (32, 32') tournée vers la première extrémité (16, 16') au niveau de la deuxième extrémité (24, 24') de la tige (22, 22') est en saillie radialement par rapport à une section centrale (40, 40') de la tige (22, 22') qui s'étend entre les extrémités (16, 24; 16', 24') de la tige (22, 22').

3. Ensemble d'instruments selon la revendication 2, **caractérisé en ce que** la surface d'introduction (32, 32') tournée vers la première extrémité (16, 16') au niveau de la deuxième extrémité (24, 24') de la tige (22, 22') est une surface annulaire.

4. Ensemble d'instruments selon l'une des revendications précédentes, **caractérisé en ce que** la surface d'introduction (32, 32') tournée vers la première extrémité (16, 16') à la deuxième extrémité (24, 24') de la tige (22, 22') est bombée.

5. Ensemble d'instruments selon l'une des revendications précédentes, **caractérisé en ce que** la deuxième extrémité (24, 24') de la tige (22, 22') présente une section d'extrémité en forme de calotte sphérique (44, 44') qui forme la surface de transmission de force (26, 26') pour introduire les forces externes en direction de la première extrémité (16, 16') de la tige (22, 22').

6. Ensemble d'instruments selon au moins la revendication 2, **caractérisé en ce que** la masse d'impulsion (14) possède pour recevoir la section centrale (40, 40') de la tige (22, 22') un évidement (52) qui présente un épaulement (54) formant la contre-surface (34) au niveau de la masse d'impulsion (14), au niveau duquel la surface d'introduction en saillie radiale (32, 32') tournée vers la première extrémité (16, 16') au niveau de la deuxième extrémité (24, 24') de la tige (22, 22') peut être amenée en butée par frappe.

7. Ensemble d'instruments selon la revendication 6, **caractérisé en ce que** la masse d'impulsion (14) est munie d'une fente (56) qui s'étend parallèlement à l'évidement (52) et par le biais de laquelle la section centrale (40, 40') de la tige (22, 22') peut être insérée dans l'évidement (52).

8. Ensemble d'instruments selon la revendication 6 ou 7, **caractérisé en ce que** la contre-surface (34) au niveau de l'épaulement (54) est formée sur un côté interne de l'épaulement (54) par rapport à l'évidement (52) et l'évidement (52) présente devant l'épaulement (54) une section transversale (55) qui est plus grande que la plus grande section transversale de la deuxième extrémité (24, 24') pour un mouvement sans entrave même de la deuxième extrémité (24, 24') de la tige (22, 22') dans l'évidement (52).

9. Ensemble d'instruments selon au moins la revendication 6, **caractérisé en ce que** l'évidement (52) pour recevoir la tige (22, 22') de l'outil d'entraînement (10, 12) est aligné avec le deuxième axe (38) de la masse d'impulsion (14) et s'étend du manche de marteau (37) dans la . tête de marteau (35) où l'évidement (52) se termine ouvert avec son épaulement (54).

10. Ensemble d'instruments selon au moins les revendications 7 et 9, **caractérisé en ce que** le manche de marteau (37) présente une ouverture de jonction (58) qui s'étend dans une direction transversale au deuxième axe (38) et jouxte la fente (56), qui se termine dans l'évidement (52) et sert à insérer la deuxième extrémité (24, 24') de la tige (22, 22') dans l'évidement (52).

11. Ensemble d'instruments selon l'une des revendications précédentes, **caractérisé en ce que** la tête de marteau (35) présente une surface de frappe plane sur des côtés opposés l'un à l'autre comme surface de transmission de force (28) possible.

12. Ensemble d'instruments selon l'une des revendications précédentes, **caractérisé en ce que** l'outil d'entraînement (10, 12) est un dilatateur pour élargir un alésage osseux (15, 15'), qui présente au niveau de sa première extrémité (16, 16') une section d'élargissement (18, 20) agencée pour un élargissement symétrique ou asymétrique de l'alésage osseux.
